# EUROPÄISCHE PATENTSCHRIFT

(11) **EP 0 998 236 B1**
(45) Veröffentlichungstag und Bekanntmachung des Hinweises auf die Patenterteilung: **31.10.2001**
(21) Anmeldenummer: 98928269.4
(22) Anmeldetag: 14.05.1998
(51) Int. Cl.: A61B 17/64, A61B 17/66

(54) **BIDIREKTIONALER UNTERKIEFERDISTRAKTOR**
BI-DIRECTIONAL RETRACTOR SET FOR LOWER JAW
ENSEMBLE ECARTEUR BIDIRECTIONNEL POUR MACHOIRE INFERIEURE

(30) Priorität: 25.07.1997 DE 19731921
(43) Veröffentlichungstag der Anmeldung: 10.05.2000
(73) Patentinhaber: ESKA Implants GmbH & Co., 23556 Lübeck (DE)
(72) Erfinder: GRUNDEI, Hans, D-23558 Lübeck (DE); HASSE, Andreas, D-23560 Lübeck (DE)
(74) Vertreter: Fuchs Mehler Weiss & Fritzsche
(86) Internationale Anmeldenummer: EP9802863
(87) Internationale Veröffentlichungsnummer: WO9904713

(56) Entgegenhaltungen:
- WO-A-97/14367
- DE-A- 19 537 023
- US-A- 5 364 396

## Beschreibung

Die vorliegende Erfindung betrifft einen bidirektionalen Unterkieferdistraktor.

Bei bestimmten schweren Mißbildungen im Unterkieferbereich werden sogenannte Osteomien erzeugt, die je nach Therapievorschlag mehr oder weniger stark auseinandergezogen, d.h. distrahiert werden, um beispielsweise ein möglichst gleichmäßiges Gesichtsbild beim Patienten zu erzeugen.

Zu diesem Zwecke sind aus der Praxis Unterkieferdistraktoren bekannt, welche aus zwei am Unterkieferknochen fixierbaren Grundplatten bestehen, welche mittels eines zylinderstiftgeführten Gebtriebemechanismus mit einer Vortriebsspindel zueinander lageveränderlich angeordnet sind. Zwischen den beiden Grundplatten befindet sich die Osteomie: der Distraktor überbrückt also die Osteomie.

Als Beispiel hierfür möge ein Distraktor gemäß der US-PS 5,364,396 dienen.

Ein ähnlich aufgebautes System ist im übrigen bekannt aus der DE-A-195 42 064, welche eine Osteosyntheseplatte beschreibt. Baugrößenbedingt ist jedoch der Einsatz einer derartigen Osteosyntheseplatte im Unterkieferbereich nicht möglich.

Ein derartiger intraoral applizierter Distraktor wird durch Ankoppelung einer von Hand zu betätigenden Welle, die an die Vortriebsspindel ankoppelbar ist, betätigt, d.h. auseinandergezogen. Zur Ankoppelung kann die Vortriebsspindel mit einem Vierkant am Distraktorausgang versehen sein und die von Hand zu betätigende Welle mit einem entsprechend ausgebildeten Innenvierkant.

Oftmals sind allerdings die Verwachsungen oder Mißbildungen so schwer, daß mindestens zwei Osteomien in dem Unterkieferknochen (Mandibula) vorgenommen werden müssen. Bei der sich dann anschließenden Distraktion ergibt sich die Schwierigkeit, das Maß der Distraktion des einen Distraktors an das Maß der Distraktion des anderen Distraktors anzupassen. Darüberhinaus ist die Handhabung eines Distraktors, der beispielsweise im Bereich des Angulus mandibulae angebracht ist, recht schwierig. Aus diesem Grunde ist der oben erwähnte bekannte Distraktor im Bereich der Ankoppelung der von Hand zu bedienenden Welle an die Vortriebsspindel mit einem Kardangelenk versehen, wodurch die Handhabung etwas erleichtert wird. Das Problem jedoch der gezielten Abstimmung der Distraktionsmaße bei mehreren Distraktoren bleibt unverändert erhalten.

Ein gattungsgemäßer bidirektionaler Unterkieferdistraktor ist bekannt aus der WO-A-97/14367. Darin wird ein bidirektionaler Unterkieferdistraktor beschrieben, bei dem beide Distraktoren gemeinsam eine Grundplatte aufweisen. Die exakte Einstellung beider Distraktoren ist in diesem Falle recht mühselig und schwierig durchzuführen.

Vor diesem Hintergrund ist es die Aufgabe der vorliegenden Erfindung, den gattungsgemäßen Unterkieferdistraktor so weiterzubilden, daß bei der Behandlung von Mißbildungen mittels zweier Osteomien eine gezielte Abstimmung beider Distraktoren ermöglicht wird, wobei die Handhabung erheblich vereinfacht werden soll.

Diese Aufgabe wird gelöst durch einen bidirektionalen Unterkieferdistraktor mit den Merkmalen des Anspruchs 1. Weitere vorteilhafte Ausgestaltungen ergeben sich aus den Unteransprüchen.

Demgemäß wird vorgeschlagen, daß bei dem bidirektionalen Unterkieferdistraktor die zwei Distraktoren jeweils mittels einer flexiblen Welle miteinander gekoppelt sind, derart, daß die Längsachsen der einzelnen Distraktoren zueinander jeweils in einem Winkel im Bereich von 0<α<90° geneigt sein können, wobei die flexible Welle die Drehung der Vortriebsspindel des frontal gelegenen Distraktors auf die Vortriebsspindel des anderen Distraktors überträgt, wobei an das frontale Ende der Vortriebsspindel des frontal gelegenen Distraktors eine abnehmbare Handspindel ankoppelbar ist. Letztere kann in bevorzugter Ausführungsform ebenfalls flexibel sein.

Mit anderen Worten besteht der erfindungsgemäße Unterkieferdistraktor aus zwei Distraktoren im wesentlichen bekannter Art, die über eine flexible Welle miteinander gekoppelt sind. Die flexible Welle gestattet eine winkelige Anordnung der einzelnen Distraktoren zueinander. Jeder Distraktor ist dabei einer künstlich anzubringenden Osteomie im Unterkiefer zuzuordnen. So ist es ohne weiteres möglich, einen Distraktor im Bereich des Angulus mandibulae anzuordnen, den anderen im frontalen Bereich des Unterkieferknochens, wobei der Winkel zwischen den Längsachsen beider Distraktoren den angegebenen Bereich einnehmen kann. An dem frontal gelegenen Distraktor kann nun die von Hand betätigbare, ggf. flexible Handspindel angesetzt werden, um die Osteomien auseinanderzuziehen oder ggfls. zusammenzuziehen. Dabei ist die Handhabung besonders einfach, da der Distraktor im Bereich des Angulus mandibulae nicht separat mittels einer Handspindel angetrieben werden muß.

Gemäß einer Ausführungsform ist vorgesehen, daß die Vortriebsspindeln Gewindespindeln mit derselben Gewindesteigung sind. Dies gewährleistet, daß die Osteomien beim Ansetzen und Drehen der ggf. flexiblen Handspindel um denselben Betrag verkürzt oder verlängert werden.

Gemäß einer anderen Ausführungsform ist vorgesehen, daß die Vortriebsspindeln Gewindelspindeln mit unterschiedlichen Gewindesteigungen sind. Diese Ausführungsform ermöglicht es, beim Drehen der eingesetzten, ggf. flexiblen Handspindeln die eine Osteomie in einem anderen Maße zu verkürzen oder zu verlängern als eine andere Osteomie. Dies kann bei bestimmten Erscheinungsbildern erwünscht sein.

Gemäß einer vorteilhaften Weiterbildung ist vorgesehen, daß die Vortriebsspindeln gleichsinnige Gewinde aufweisen. Hier bewirkt die Betätigung der eingesetzten (flexiblen) Handspindel, daß die Grundplatten jedes Distraktors örtlich im gleichen Sinne verlagert werden, d.h. daß die Osteomien gleichzeitig verkürzt oder aber ggfls. verlängert werden.

In einer anderen Ausführungsform ist vorgesehen, daß die Vortriebsspindeln gegensinnige Gewinde aufweisen, also beispielsweise eine Vortriebsspindel ein Rechtsgewinde, die andere ein Linksgewinde. Damit kann erreicht werden, daß beispielsweise die eine Osteomie auf Betätigung der angesetzten ggf. flexiblen Handspindel verlängert, eine andere Osteomie jedoch verkürzt wird, in Abhängigkeit davon, welcher Distraktor über welche Osteomie gesetzt worden ist.

Alle Ausführungsformen können dadurch vorteilhaft weitergebildet werden, indem im Bereich der flexiblen Welle Winkelstücke zwischen den jeweils benachbarten Grundplatten der einzelnen Distraktoren mit jeweils zwei einen Winkel 0 < α < 90° bildenden Schenkeln eingesetzt und an diesen befestigt sind. Mit anderen Worten wird der Bereich zwischen zwei Grundplatten zweier benachbarter Distraktoren, in welchem die flexible Welle verläuft, überbrückt durch Winkelstücke. Dies erhöht die Stabilität der winkeligen Anordnung der Distraktoren zueinander erheblich. Der Winkel wird präoperativ beispielsweise anhand von Röntgenbildern ermittelt und die mindestens zwei Distraktoren werden vor der Operation mit den geeignet erscheinenden Winkelstücken vormontiert, so daß auf diese Art und Weise eine mechanisch stabile Implantationseinheit in Form eines Distraktorenzuges erstellt wird und zum Einsatz kommt. Der Arzt kann präoperativ sehr präzise ein Implantat erstellen, welches den Erfordernissen des jeweiligen individuellen Erscheinungsbildes des Patienten am besten gerecht wird.

Die Erfindung wird anhand der Zeichnungsfiguren beispielhaft näher erläutert. Hierbei zeigt:
- Figur 1: zwei aneinander gekoppelte Distraktoren,
- Figur 2: zwei aneinander gekoppelte Distraktoren mit abgewinkelter flexibler Welle, und
- Figur 3: die Aufsicht auf ein Winkelstück zum Einsatz zwischen zwei Distraktoren.

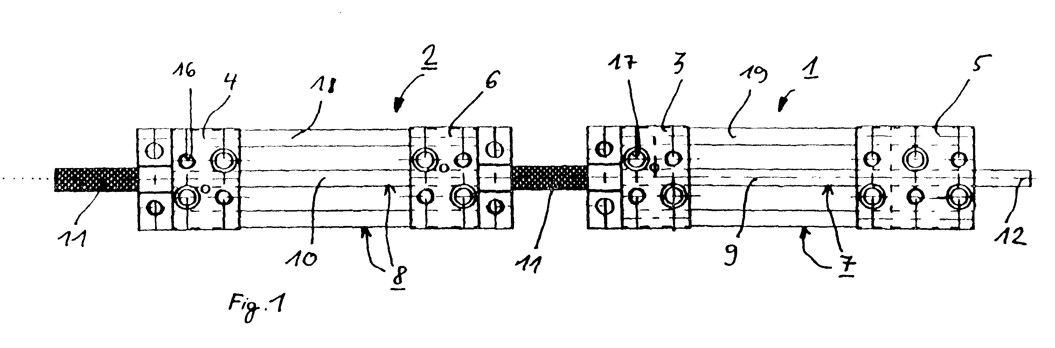

In Figur 1 ist die Ansicht zweier aneinandergekoppelter Distraktoren 1 und 2 dargestellt. Jeder Distraktor 1,2 besteht aus zwei Grundplatten 3,5 bzw. 4,6. Diese Grundplatten weisen Bohrungen 16 und 17 auf, durch welche hindurch geeignete Knochenschrauben gesetzt werden können, damit die Distraktoren am Unterkieferknochen befestigt werden können.

Jeder Distraktor weist einen Getriebemechanismus 7 bzw. 8 auf, der, wie im vorliegenden Fall dargestellt, ein zylinderstiftgeführter Getriebemechanismus sein kann. Die Zylinderstifte 18 bzw. 19 dienen zur Führung, wohingegen die Vortriebsspindeln 8 bzw. 9 für ein Auseinanderziehen bzw. Zusammenziehen des Distraktors sorgen. Dies geschieht dadurch, daß die Vortriebsspindeln 9 bzw. 10 Gewindespindeln sind und dadurch, daß an das frontale Ende 12 des frontal gelegenen Distraktors 1 eine abnehmbare, flexible Handspindel koppelbar ist und diese gedreht wird. Die Drehung der Vortriebsspindel 9 wird auf die Vortriebsspindel 10 des anderen Distraktors 2 dadurch übertragen, daß beide Distraktoren 1 und 2 miteinander gekoppelt sind durch eine flexible Welle 11.

Die Osteomien werden überbrückt durch die Distraktoren 1 und 2, d. h., daß zwischen den Grundplatten 3 und 5 des Distraktors 1 sowie zwischen den Grundplatten 4 und 6 des Distraktors 2 eine Osteomie im Unterkieferknochen vorgenommen wird.

Die Vortriebsspindel 9 kann vorliegend beispielsweise ein Rechtsgewinde aufweisen, die Vortriebsspindel 10 des anderen Distraktors 2 beispielsweise ein Linksgewinde. Auf die Drehung einer am frontalen Ende 12 des Distraktors 1 angekoppelten, flexiblen Handspindel hin wird daraufhin der eine Distraktor verkürzt und der andere Distraktor verlängert. Individuell kann der Arzt vor der Implantation durch Auswahl bestimmter Vortriebsspindeln die Therapie vorplanen und den Distraktorzug zusammensetzen. Parameter dabei sind die Steigung des Gewindes sowie eine etwaige Gegensinnigkeit der Gewinde.

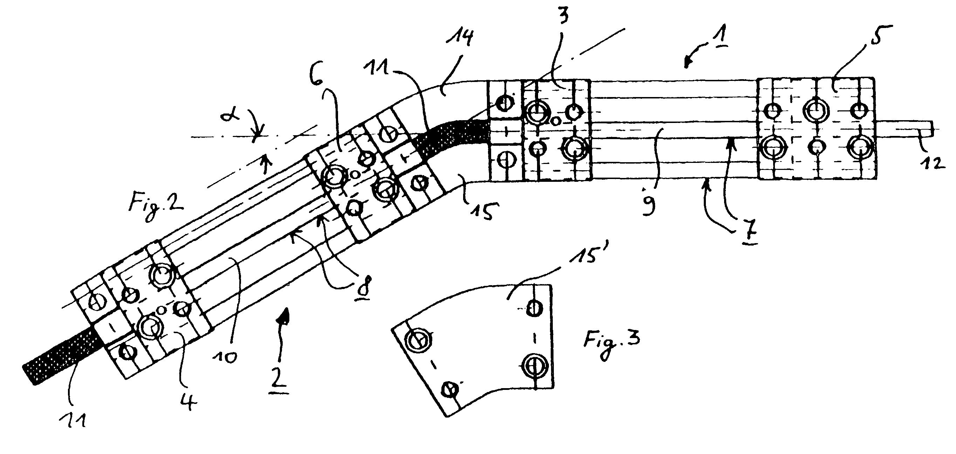

Figur 2 zeigt den erfindungsgemäßen Unterkieferdistraktor mit abgewinkelter flexibler Welle 11, so daß die Längsachsen der Distraktoren 1 und 2 einen Winkel α zwischen sich einschließen, der im Bereich von 0 bis 90° liegen kann.

Das Implantat wird dadurch stabilisiert, daß in dem Bereich der flexiblen Welle zwei Winkelstücke 14 und 15 eingesetzt sind, die mit den benachbarten Grundplatten 3 und 6 verschraubt werden können.

Figur 3 zeigt eine andere Ausführungsform des Winkelstückes 15', welches die beiden Winkelstücke 14 und 15 bei dem Distraktor in Figur 2 ersetzt.

Durch Auswahl der Winkelstücke wird das Implantat vor der Operation stabilisiert und es kann im vormontierten Zustand eingesetzt werden.

Das Implantat wird am Unterkieferknochen durch Knochenschrauben fixiert. Der Operateur ist nun in der Lage, durch Ankoppeln einer (flexiblen) Handspindel an das frontale Ende 12 des frontal gelegenen Distraktors 1 die vorgeplanten Verkürzungen und/oder Verlängerungen der Osteomien vorzunehmen.

Der erfindungsgemäße Distraktor ist außerordentlich vielseitig und läßt sich individuell an die jeweiligen Bedürfnisse anpassen, wobei zu bemerken ist, daß er aus gleichartigen Standardteilen zusammengefügt wird.

## Patentansprüche

1. Bidirektionaler Unterkieferdistraktor, aufweisend zwei Distraktoren (1, 2) mit jeweils zwei am Unterkieferknochen fixierbaren Grundplatten (3, 5; 4, 6), die mittels eines Getriebemechanismus (7, 8) mit jeweils einer Vortriebsspindel (9, 10) zueinander lageveränderlich angeordnet sind,
**dadurch gekennzeichnet,**
**daß** die beiden Distraktoren (1, 2) jeweils mittels einer flexiblen Welle (11) miteinander gekoppelt sind, derart, **daß** die Längsachsen der einzelnen Distraktoren (1, 2) zueinander jeweils in einem Winkel im Bereich von 0<α<90° geneigt sein können, wobei die flexible Welle (11) die Drehung der Vortriebsspindel (9) des frontalgelegenen Distraktors (1) auf die Vortriebsspindeln (10) des anderen Distraktors (2) überträgt, wobei an das frontale Ende (12) der Vortriebsspindel (9) des frontal gelegenen Distraktors (1) eine abnehmbare Handspindel ankoppelbar ist.

2. Unterkieferdistraktor nach Anspruch 1, bei dem die Vortriebsspindeln (9,10) Gewindespindeln mit derselben Gewindesteigung sind.

3. Unterkieferdistraktor nach Anspruch 1, bei dem die Vortriebsspindeln (9,10) Gewindespindeln mit unterschiedlichen Gewindesteigungen sind.

4. Unterkieferdistraktor nach einem der Ansprüche 1 bis 3, bei dem die Vortriebsspindeln gleichsinnige Gewinde aufweisen.

5. Unterkieferdistraktor nach einem der Ansprüche 1 bis 3, bei dem die Vortriebsspindeln gegensinnige Gewinde aufweisen.

6. Unterkieferdistraktor nach einem der Ansprüche 1 bis 5, bei dem im Bereich der flexiblen Wellen (11) Winkelstücke (14,15,15') zwischen den jeweils benachbarten Grundplatten (3,6) der einzelnen Distraktoren (1,2) eingesetzt und an diesen befestigt sind.

7. Unterkieferdistraktor nach einem der Ansprüche 1 bis 6, bei dem die Handspindel als flexible Welle (11) ausgebildet ist.

## Claims

1. Bidirectional lower jaw retractor having two retractors (1, 2) each having two base plates (3, 5; 4, 6) which can be fixed to the lower jaw bone and which are arranged to change the position with respect to one another by means of a gear mechanism (7, 8) having in each case an advancing spindle (9, 10), **characterised in that** the two retractors (1, 2) are coupled to one another in each case by means of a flexible shaft such that the longitudinal axes of the individual retractors (1, 2) may be inclined with respect to one another in each case at an angle in the range from 0<α<90°, wherein the flexible shaft (11) transfers the rotation of the advancing spindle (9) of the frontally placed retractor (1) to the advancing spindles (10) of the other retractor (2), wherein a removable hand spindle can be coupled to the frontal end (12) of the advancing spindle (9) of the frontally placed retractor (1).

2. Lower jaw retractor according to claim 1, in which the advancing spindles (9, 10) are threaded spindles having the same thread pitch.

3. Lower jaw retractor according to claim 1, in which the advancing spindles (9, 10) are threaded spindles having different thread pitches.

4. Lower jaw retractor according to one of claims 1 to 3, in which the advancing spindles have threads running in the same direction.

5. Lower jaw retractor according to one of claims 1 to 3, in which the advancing spindles have threads running in opposite direction.

6. Lower jaw retractor according to one of claims 1 to 5, in which in the region of the flexible shafts (11), angle plates (14, 15, 15') are inserted between in each case neighbouring base plates (3, 6) of the individual retractors (1, 2) and are attached to the latter.

7. Lower jaw retractor according to one of claims 1 to 6, in which the hand spindle is designed as a flexible shaft (11).

## Revendications

1. Ensemble écarteur bidirectionnel pour mâchoire inférieure, présentant deux écarteurs (1, 2) comprenant respectivement deux plaques de base (3, 5 ; 4, 6) pouvant être fixées au maxillaire inférieur, qui, grâce à un mécanisme d'entraînement (7, 8) peuvent être disposées de façon à être déplacées, avec respectivement une broche d'avancement (9, 10),
**caractérisé par le fait que**
les deux écarteurs (1, 2) sont couplés respectivement au moyen d'une tige flexible (11), de sorte que les axes longitudinaux des écarteurs respectifs (1, 2) puissent être inclinés l'un vers l'autre en un angle compris entre 0<a<90°, la tige flexible (11) transmettant le mouvement de rotation de la broche d'avancement (9) de l'écarteur frontal (1) à la broche d'avancement (10) de l'autre écarteur (2), une broche à vis manuelle amovible pouvant être couplée à l'extrémité frontale (12) de la broche d'avancement (9) de l'écarteur frontal (1).

2. Ensemble écarteur selon la revendication 1, dans lequel les broches d'avancement (9, 10) sont des broches filetées avec le même pas de vis.

3. Ensemble écarteur selon la revendication 1, dans lequel les broches d'avancement (9, 10) sont des broches filetées avec des pas de vis différents.

4. Ensemble écarteur selon les revendications 1 à 3, dans lequel les broches d'avancement ont des filetages de même sens.

5. Ensemble écarteur selon les revendications 1 à 3, dans lequel les broches d'avancement ont des filetages de sens contraires.

6. Ensemble écarteur selon les revendications 1 à 5, dans lequel, dans la région des tiges flexibles (11), des raccordements angulaires (14, 15, 15') sont placés entre les plaques de base adjacentes respectives de chaque écarteur (1, 2) et y sont fixés.

7. Ensemble écarteur selon les revendications 1 à 6, dans lequel la broche à vis est conçue comme tige flexible (11).
